# EUROPEAN PATENT SPECIFICATION

(11) **EP 1 707 192 B1**
(45) Date of publication and mention of the grant of the patent: **25.02.2009**
(21) Application number: 06111360.1
(22) Date of filing: 17.03.2006
(51) Int. Cl.: A61K 9/16

(54) **Pharmaceutical composition**
Pharmazeutische Zusammensetzung
Composition pharmaceutique

(30) Priority: 30.03.2005 IT MI20050517
(43) Date of publication of application: 04.10.2006
(73) Proprietor: THERAPICON SRL, I-20146 Milan (IT)
(72) Inventor: Veronesi, Paolo Alberto, 20146 Milan (IT); Veronesi, Anna Maria, 20146 Milan (IT)
(74) Representative: Rickard, David John

(56) References cited:
- EP-A- 0 711 560
- FR-A- 2 668 705

## Description

The present invention relates to pharmaceutical compositions containing flavonoids for oral or topical administration routes. The invention also concerns the preparation process to yield a solid pharmaceutical composition characterized to exhibit an extraordinary wettability and improved bioavailability and to formulate a topical preparation free from the unpleasant "coarse" effect.

It has been already disclosed in many publications for example: Martindale 33^{rd} Ed. page 1611; Vidal 75^{th} Ed., pages 348, 388, 476, 488, 576, 577, 661, 745, 1155, 1530, 2013; Merck Index 13^{th} Ed. entries 326, 3327, 4690 and 5520, that purified flavonoids, like diosmin, hesperidin and other naturally occurring flavonic glycosides, such as isorhoifolin, linarin, diosmetin, individually or in combination, are used in medicine for the treatment of chronic venous diseases. In fact, flavonoids are capillary protectants and exhibit venotonic and vasculoprotective properties. The daily intake may generally vary from 400 to 2000 mg, divided into 2-3 daily administrations.

Since flavonoids, like diosmin, hesperidin, linarin, diosmetin, quercitin, rutin and other flavonic glycosides, are practically insoluble in water (see Merck Index 13^{th} Ed., entries 3326, 3327, 4690, 5520, 8118, 8382, Eur. Ph. 5th Ed. page 1452), when a systemic medicinal effect is desired, they are administered solely by oral route.

Thus they are generally formulated in the conventional pharmaceutical forms of tablets and granules. Topical preparations are also used for haemorrhoids (Martindale 33^{rd} Ed. page 1611.2). However, the most widely used flavonoid, namely diosmin, and other flavonoids as well, are hygroscopic substances, see Eur. Ph. 5th Ed. page 1452 and also, EP 0711560 (page 2, lines 50-51) which indicates that diosmin contains between 4 and 6 % of water.

Several difficulties are experienced when compounding these substances into solid forms of medicinal specialities; firstly, in view of the large mass of flavonoids contained in each dose unit and secondly, due to their known insolubility in water. In fact, the copious bibliographic references underline that these are the two major technical questions to be solved particularly when formulating solid compositions of flavonoids. Patent FR 2661610 describes a novel pharmaceutical lyophilised composition to be disintegrated in water and administered as a drinkable preparation. This composition teaches lyophilised diosmin in a percentage between 30 % and 80 %, more preferably between 40 % and 80 % of the dry mass, a diluent up to 60 % of the dry mass and several other inert ingredients, like suspending agents, additives, colouring agents and optionally a surface agent. However, this lyophilised composition appears to be rather unsuitable, since the lyophilisation of diosmin is a long and expensive process, especially when large amounts of diosmin have to be processed.

In addition, patients often report that they experience an unpleasant taste when taking flavonoids formulated in this kind of drinkable preparation. This second disadvantage can lead to therapeutic failures, since frequently the patient drops the treatment, while the venous insufficiency is a chronic disease requiring a continuous treatment during several months or even years, without any interruptions. In an attempt to overcome the problem of unpleasant taste, WO 2004/032942 describes a pharmaceutical chewable tablet containing diosmin or alternatively granulated diosmin, mixed with auxiliary excipients already known in the art. An embodiment of this prior art invention further teaches that the active ingredient diosmin contained in the tablet of the invention shall have a moisture content of between 1 % and 5 %, more preferably between 2 % and 4 %, and that the particle size shall be in the range from 20 to 50 microns. In another embodiment, the active ingredient contained in the tablet is granulated diosmin, having a moisture content of between 4 % and 8 %, and a particle size within an interval of from 50 to 150 microns. The main disadvantage of the above formulation is that the total mass of the chewable tablet is extremely large, in a range from 2.0 to 3.0 grams for each unit dose, due to the massive use of large amounts of auxiliary excipients aiming to improve the taste of the composition.

More recently, EP 0541874 describes pharmaceutical preparations to produce a solution or a fine suspension of diosmin, where the grain size of diosmin lies for at least 90 % between 100 and 400 microns. This patent also describes the use of significant amounts of a copolymer of ethylene oxide and propylene oxide, commercialised under the trademark "Plurionic F68^{®"}, and also of povidone or polyethylene glycol for the preparation of powder for sachets and tablets. Nevertheless this patent does not explain either how it can produce a "solution of diosmin" (diosmin is rather insoluble in water) or provide any indication as to the precise galenical advantages derived from the claimed composition. Another patent, EP 0711560 describes an effervescent granulate in the form of a tablet or a sachet comprising diosmin in micronized form presenting a granule size from 0.1 to 10 microns. However, this patent indicates that diosmin has a moisture content of between 4 % and 6 % and that this water represents a considerable inconvenience for the effervescent composition. In fact, the present Applicant believes, the simultaneous use of large amounts of bicarbonate, sodium bicarbonate and considerable quantities of citric acid (as in the Examples from 1 to 4) may generate, in the presence of moisture, the spontaneous effervescence of the granulate, so that the moisture content does not allow a good stability of the claimed composition (page 2, lines 51-55).

Moreover to overcome this galenical inconvenience an original monophasic process is also described, in which the granulate is produced without the addition of external water, but by adding a minimal quantity of ethanol (page 2, lines 54-58 and page 3, lines 1-2). However, such a process also presents technical difficulties, since the use of organic solvents, like ethanol, for the granulation process will require the availability and use of expensive antideflagrating equipments. Moreover this patent indicates that it is compulsory that, after drying, the moisture content of the granulate shall be less than 0.5 %, the condition at which the effervescent granulate of the invention is stable (page 3 lines 2-3). Additionally this prior art specifies that the pharmaceutical composition does not contain either a tensioactive agent or a thickening agent (page 3, lines 7-8). The high weight (5000 mg) of the unit dose of the claimed composition is also very unusual. Moreover, in the pharmaceutical form of effervescent tablet or sachet, the active ingredient (1000 mg) represents only 20 % of the total mass of the finished composition.

Thus, none of the above patents seem to have solved efficiently the galenical problems to formulate solid medicinal products containing flavonoids. In fact, the present inventors believe that the proposed solutions are inadequate, insufficient and contradictory. In particular, it is unclear at which particle size the flavonoids are to be used, also the high amounts of auxiliary substances and excipients lead to pharmaceutical forms of extremely large size and with unpleasant taste, as with the drinkable preparation, and these are definitely not suitable for long term treatments.

Moreover, the technical solutions of the prior art show important limitations and significant disadvantages, such as an expensive lyophilisation process for the active ingredient diosmin, the instability of the effervescent granulate in presence of moisture, the use of ethanol and of antideflagrating equipments for the proposed granulation process with ethanol, to mention but some of them.

Other conventional formulations and general techniques for the preparation of pharmaceutical compositions for oral administration are summarized and may be easily found in specialized publications, as for instance Remington's Pharmaceutical Sciences (Mack Publishing Co., Easton, Pa.). Nevertheless these conventional teachings do not achieve any satisfactory results for the preparation of solid oral compositions of flavonoids, due to different reasons, such as large size of the resulting tablet, excessive friability or hardness, scarce porosity and wettability, unsatisfactory disaggregation, scratching or irregular surface.

In comparison with the above, only minor compounding problems are faced when the flavonoids are formulated as a preparation for topical use, but even here, it is still difficult to maintain homogeneity in a conventional flavonoids preparation and the "coarse" effect when it is applied to the skin or to the mucosa, is an inconvenience well known to skilled personnel.

### GENERAL

In view of the above unsatisfactory results described in the prior art, the present inventors have studied a new approach to overcome and to solve the reported galenical problems particularly related to the preparation of suitable solid formulations of said flavonoids.

The present invention relates generally to pharmaceutical compositions for oral or topical administration comprising microcrystalline flavonoids. In one embodiment the pharmaceutical composition can be made in granular form, either with or without other inert or auxiliary ingredients, and these granules may be compacted to form a solid formulation suitable for oral administration. The process for the formation of the granules may significantly contribute to yield the final quality of the solid oral pharmaceutical composition of the invention, which exhibits an good wettability and a good absorption profile. In one embodiment of the invention the microcrystalline flavonoids within a pharmaceutical composition are preferably formulated as a topical composition that, in addition to a high wettability, does not present the "coarse" effect when applied to the skin or to the rectal mucosa.

In one embodiment the present invention provides a pharmaceutical composition comprising an active ingredient comprising two or more flavonoids in microcrystalline form, wherein the two or more microcrystalline flavonoids comprise diosmin and at least one other flavonoid; and further wherein the two or more microcrystalline flavonoids exhibit the following characterizing parameters:
(a) a specific surface area of the microcrystalline flavonoids of from 4 to 18 m²/g, preferably 9 to 12 m²/g, according to BET determination;
(b) an F/D (F = other flavonoids and D = diosmin) (weight/weight) ratio of from 0.03 to 0.1; and
(c) a moisture content of less than 4.0 % by weight.

In a further embodiment of the present invention the two or more microcrystalline flavonoids exhibit a typical X-ray powder ray diffraction pattern with peaks of relative intensity "strong" (s) at about 15.32, 19.68 and 21.70, "medium" (m) at 11.90, 15.87, 21.33 and 24.68 and diffuse (d) at about 26.35, 27.72 and 28.65.

In a further embodiment of the present invention there is provided a pharmaceutical composition in solid form, for example a tablet or granule, which is suitable for oral administration, wherein the amount of the two or more microcrystalline flavonoids represents not less than 70.0 % (w/w) of the total mass of the solid.

In a further embodiment of the present invention there is provided a topical preparation, for example a fatty cream or ointment for external topical use, wherein the amount of the two or more microcrystalline flavonoids represents not less than 4.0 % (w/w) of the weight of the topical preparation.

In a further embodiment of the present invention a solid pharmaceutical composition in tablet or granule form further comprises a disintegrating substance. Preferably said disintegrating substance is not less than 15.0 % (w/w) of the total mass of the inactive or auxiliary ingredients. One suitable disintegrating substance is sodium starch glycolate but others known in the art may be used.

Preferably tablets or granules according to an embodiment of the present invention may be obtained by means of a wet granulation process spraying or wetting or kneading the two or more microcrystalline flavonoids and selected inert (inactive) and auxiliary ingredients with a suitable amount of water in a typical characterizing sequence.

Preferably the inert and auxiliary ingredients are present in quantities suitable to yield a physiological range of pH comprised between 6.5 and 7.5.

It is particularly preferred that tablets or granules according to the present invention have a water content more than 2.0 % (w/w).

The two or more flavonoids in microcrystalline form used in the present invention preferably comprise diosmin as the dominant microcrystalline flavonoid component. Preferably, the diosmin makes up more than 50% by weight and further preferably more that 90% by weight of the two or more microcrystalline flavonoids.

Preferably at least one other flavonoid may be selected from one or more of isorhoifolin, linarin, diosmetin, hesperidin, quercitin and rutin.

A pharmaceutical composition according to one embodiment of the invention may comprise two or more microcrystalline flavonoids obtained from separate sources and then combined in the desired ratio. Alternatively, any suitable reaction process may be used which gives a reaction product comprising microcrystalline diosmin and one or more other microcrystalline flavonoids; for example, a known reaction process involves the reaction of hesperidin and iodine/potassium acetate in methanol. When such reaction processes are used, the dominance of diosmin in the reaction product may be suitably enhanced by any technique known in the art; for example, fractioned precipitation in water at a pH of 5 to 6. Once formed, the mixture of two or more microcrystalline flavonoids can then be dried, preferably with vigorous shaking in a vacuum dryer at a temperature of 75°C or above, and then milled to ensure that the specific surface area is from 4 to 18 m²/g, preferably 9 to 12 m²/g, according to BET determination.

In yet anther embodiment there is provided a process comprising the following production sequence:
(a) incorporate homogeneously microcrystalline cellulose into two or more microcrystalline flavonoids (powder mixture);
(b) prepare separately a binding solution by dissolving gelatine, glycerin and sodium lauryl sulfate in a convenient quantity of water;
(c) conveniently spray or wet or knead a powder mixture (a) with the binding solution (b) to yield the wet granulate to be conveniently sieved into granules of adequate consistency and size;
(d) dry the wet granulate on a fluid-bed desiccator (moisture more than 2.0 %) to be conveniently milled and sieved; and
(e) homogeneously blend the dry granules from (d) with sodium starch glycolate to yield the compression mixture to be punched into tablets.

The microcrystalline flavonoids formed by processes described herein may comprise a mixture of monohydrate and dihydrate microcrystalline forms.

Preferably, the monohydrate form is the major component, further preferably the monohydrate form is present in an amount of more than 50% by weight, an amount of 60% by weight of the flavonoids mixture being especially preferred.

The preferred two or more microcrystalline flavonoids used in the pharmaceutical composition of the present invention exhibit typical "X-ray powder diffractograms (XRD)", as that shown in Figure N. °1, where the XRD of a specific production batch has been traced. Typical X-ray diffractograms of other production batches of microcrystalline flavonoids are additionally shown in Figure N.°2. The typical X-ray powder diffraction patterns of the preferred two or more microcrystalline flavonoids used in the present invention exhibit relative intensity peaks "strong" (s) at about 15.32, 19.68 and 21.70, "medium" (m) at 11.90, 15.87, 21.33 and 24.68 and diffuse (d) at about 26.35, 27.72 and 28.65.

Preferably in one embodiment a pharmaceutical composition according to the invention is present in the form of granulate to fill monodose sachets, characterized in that it is prepared by admixing the granules formed according to a prcess as described herein with suitable amounts of solid sweetening agents and flavouring agents.

In yet a further embodiment there is provided a process for the preparation of a pharmaceutical composition in the form of fatty cream or ointment comprising admixing 4.0 % (w/w) of the two or more microcrystalline flavonoids with convenient quantities of suitable inert and auxiliary ingredients to yield a physiological range of pH comprised between 6.5 and 7.5.

In a preferred embodiment of the present invention, there is provided a reaction process for the preparation of two or more microcrystalline flavonoids, wherein the two or more microcrystalline flavonoids comprise diosmin and at least one other flavonoid, said reaction process comprising the steps:
a) reacting hesperidin with iodine/potassium acetate in methanol;
b) increasing the amount of diosmin in the reaction product obtained from step a) by fractioned precipitation; and
c) drying the reaction product obtained from step b).

According to yet a further embodiment there is provided a medicament comprising two or more microcrystalline flavonoids according to the any of the preceding claims characterized in that the medicament may be formulated as tablet, film-coated tablet, granule and granulate for oral administration, or formulated as fatty cream or ointment for external topical use, said medicament exhibiting a remarkable patient compliance and an improved wettability, absorption rate and bioavailability. Such a medicament may comprise two or more microcrystalline flavonoids in accordance with the description herein wherein the medicament exhibits venotonic and vasculoprotective effects, particularly useful for the treatment of chronic venous disorders.

### Methods and equipment used to analyse the pharmaceutical composition of the present invention.

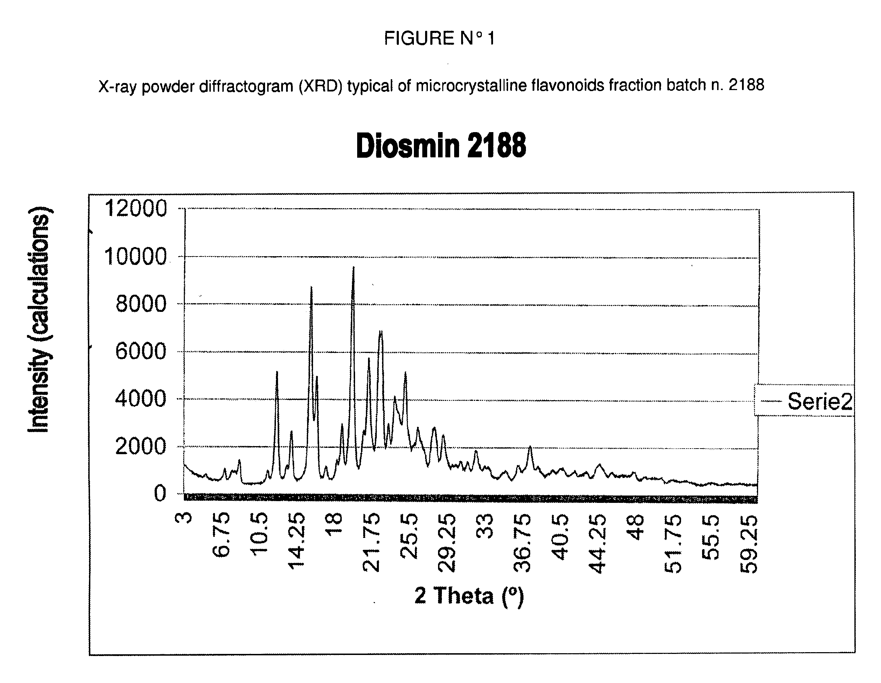

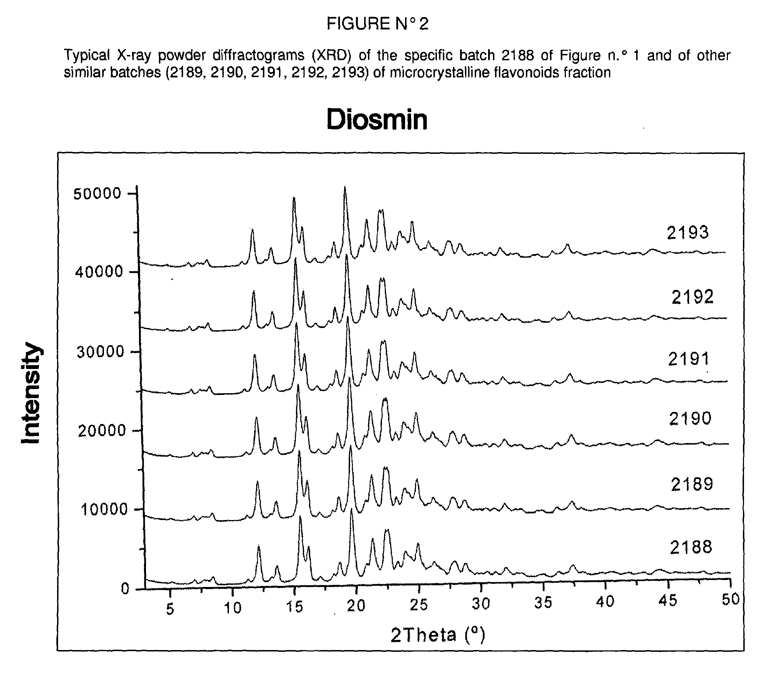

1) The "X-ray powder diffractograms (XRD)" (also cited as "diffraction patterns") of microcrystalline flavonoids can be determined with the known procedures by means of an X-ray diffractometer with Cu K alpha-1 radiation source, as for instance a system type Bruker, model AXS, D8 Advance X-ray diffractometer or a system type Seifert model XRD 3003 TT using similarly a copper target X-ray tube and the sample placed in a pyrex glass holder;
2) The "mass" of each component of the F/D ratio of the two or more microcrystalline flavonoids can be determined according to the Liquid Chromatography (Method 2.2.29) for the quantitative assay of diosmin (D) and of other flavonoids/related substances (F), published in Eur. Ph. 5th Ed. Monograph 01/2005:1611 page 1452.
3) The "specific surface area" can preferably be measured by the gas absorption method (BET method), according to S. Brunauer : "The Adsorption of Gases and Vapours", Princetown, 1945. The experimental method used to determine the specific surface area of the two or more microcrystalline flavonoids used in the pharmaceutical composition of the present invention is summarized as follows: The equipment used is a Sorptometer Kelvin 1042, which analyses the sample using a gas flow consisting of mixtures of nitrogen and helium. The above equipment generates gas mixtures at known concentrations, using gas from cylinders certified for gas-chromatography. The sample is prior heated under helium flow (pre-treatment phase), in order to eliminate from the sample surface the residual traces of the atmospheric gas mixture; the atmospheric gas mixture covering the surface of the sample may record a lower value of the surface area.
   The pre-treatment of the sample is carried out through a temperature increase, the use of vacuum, the use of helium flow or alternatively the above combined techniques, as indicated hereby :
   temperature + vacuum;
   temperature + helium (Kelvin Sorptometer).
   The use of vacuum allows a more efficient pre-treatment, but sometimes it can modify the sample, if it is sensitive to vacuum. The calculation of the figure of the specific surface area is carried out by calculating the quantity of nitrogen absorbed by the sample on its surface; nitrogen coats the sample surface as a monolayer and consequently allows the determination of a value for the total surface area.
4) The "moisture" (also cited as "water content") is determined according to the semi-micro method described in Eur. Ph. 5th Ed. Method 2.5.12 page 130.
5) The "weights" (masses) of the two or more microcrystalline flavonoids and of the inert and auxiliary ingredients (including the water to be used for the wet granulation process) are determined by means of a technical balance commonly used in a production unit of a pharmaceutical laboratory.

In order to better appreciate the pharmaceutical composition of the invention, the listed "parameters characterizing the invention" will be better illustrated here below. More particularly, the remarkable improvements of the two or more microcrystalline flavonoids used in the pharmaceutical composition of the present invention and its parameters, the wet granulation production process, the final specifications and properties of the pharmaceutical composition of the invention are better underlined in the following subsequent paragraphs.

In view of the above, the essential features of the pharmaceutical composition of the invention are:

### a) Ratio F/D (Flavonoids/Diosmin)

The preferred ratio F/D (weight/weight) between the total weight of the other flavonic glycosides (hesperidin, isorhoifolin, linarin, diosmetin) and the total weight of diosmin is comprised from 0.03 to 0.1. The preferred ratio F/D is results directly from the production process described above.

### b) Specific surface area

The specific surface area is a particularly important factor for insoluble substances as flavonic glycosides, like diosmin, hesperidin, isorhoifolin, linarin, and diosmetin.

The specific surface area is the most direct expression of the surface (expressed in square meter per gram, more simply m²/g) developed for each gram of substance and represents the area potentially available to adhere to the water during the dissolution process of a solid pharmaceutical form. Therefore, the specific surface area of an insoluble substance is the most suitable expression of both its "wettability" and "porosity". In fact, higher is the figure of the specific surface area developed by each gram of substance, higher will be the dispersion rate in water of that insoluble substance. The two or more microcrystalline flavonoids used in the pharmaceutical composition of the present invention, when obtained directly from the abovementioned reaction process, exhibit a typical specific surface area of between 4 m²/g and 8 m²/g. This product is then ground to obtain a mixture of two or more microcrystalline flavonoids with the preferred specific surface area comprised from 9 m²/g to 18 m²/g. The grinding operation can be effected with conventional equipment and methods, such as with pin disk mills or hammer mills or better with fluid-energy mills in presence of an inert gas, for example nitrogen, helium or argon. Microcrystalline flavonoids with the desired specific surface area can be obtained by varying the speed of the mill; the amount of product fed in and/or the grinding period, the force of impact and the quantity of inert gas admixed to the product. If microcrystalline flavonoids with a relatively high specific surface area are desired, as for instance of about 11 m²/g, it is advantageous to carry out grinding with a fluid-energy mill.

However, by combining the initial specific surface area and by selecting the grinding equipment, method and conditions, it is possible to obtain microcrystalline flavonoids with the desired specific surface area, suitable to prepare the pharmaceutical preparation of the invention.

Moreover the specific surface area of the microcrystalline flavonoids fraction is also particularly important for improving the bioavailability in humans, following the oral administration of one single dose of the pharmaceutical composition. The bioavailability of a drug administered by the oral route depends on its ability to be absorbed by the digestive tract. The main absorption mechanism is mainly a passive diffusion, which requires an amphophylic structure of the active ingredient in an aqueous solution. Consequently drugs that are absorbed in this way shall dissolve in the digestive fluids before diffusion through the digestive tract. The amount of drug absorbed depends on its solubility characteristics and it is determined by the amount dissolved.

The bioavailability of a drug is classically defined as the aliquot of the administered dose that reaches the systemic circulation of a living organism, and the rate at which it does so. In the specific case of the pharmaceutical composition of the invention the specific surface area is important because it is the main parameter dictating the rate of dissolution.

Noyes and Witney's law (Remington: The Science and Practice of Pharmacy, 2000, 20th Ed., P 654) allows the evaluation of this parameter:
- Dc/Dt =: KaS(Cs-Ct), where :
- Dc/Dt =: dissolution rate of the drug;
- Ka =: constant depending on the dissolution conditions;
- S =: area of contact between the powder and the solvent;
- Cs =: concentration of the drug at saturation;
- Ct =: concentration of the amount of dissolved drug at the time of measurement.

A remarkable contribution to the dissolution rate can be therefore achieved by increasing S, which is inversely proportional to the extent of milling of the powder (Chaumeil JC "Micronization: a method of improving the bioavailability of poorly soluble drugs" Meth. Find. Clin. Pharmacol. 20 (3): 211-215, 1998).

In fact, pharmacokinetic studies carried out on healthy volunteers by using the solid pharmaceutical composition of the invention, have surprisingly shown that larger is the specific surface area, higher is the absorption of the two or more microcrystalline flavonoids, thus resulting a surprising improvement in the bioavailability of the tablet of the invention.

Moreover, the consequent high value of the volume of distribution (Vd=62.02 Lt) yields an extensive uptake by the wall of the vascular tissues, wherein the microcrystalline flavonoids fraction elicits its phlebotonic activity. In this connection refer also to the publications concerning equivalent preparations: Cova D. et al., Int. J. Clin. Pharm. Ther. Tox., Vol. 30; No. 1, pages 29-33 (1992) and Garner R. C. et al., J. Pharm. Sci., Vol. 91, No. 1, pages 32-40 (2002).

### c) Water content (moisture)

The two or more microcrystalline flavonoids used in the pharmaceutical composition of the present invention are also characterized in that they are stabilized with a water-content (moisture) less than 4.0 %.

In addition to the above essential features, other features have been found to significantly influence the solid pharmaceutical composition of the invention; their combination remarkably enhances the entire process and the final quality of the pharmaceutical composition.

These other features include the amount of the flavonoids in the composition according to the invention present in tablets, granules or topical preparations, the amount of disintegrating substance, the use of a wet granulation process and the fact that the final moisture content of the pharmaceutical composition is preferably more than 2% but may be less than 2 %.

In fact, the preferred tablet of the invention exhibits from one side a reduced size, so that it can be swallowed more easily, but also a satisfactory disintegration profile, requirements that can be achieved solely by combining the above mentioned production improvements. Therefore said wet granulation process described above shall be used to admix the inert ingredients in such a proportion that the total mass of the solid pharmaceutical composition shall contain not less than 70 % (w/w) of the two or more microcrystalline flavonoids and that the total inert mass of the solid composition shall contain not less than 15 % (w/w) of disintegrating substance, while the residual water (moisture) both in the dried granules and in the final pharmaceutical composition shall be more than 2 %.

In another embodiment the preferred disintegrating substance is sodium starch glycolate.

The published literature suggests that the prescribed amounts of flavonoids for oral administration shall be comprised between 300 and 2000 mg, more preferably between 400 and 850 mg, and that the most currently used dose unit shall be formulated in the dosages of 300 mg, 400 mg, 450 mg, 500 mg and 600 mg of flavonoids. Moreover, the conventional pharmaceutical solid forms of flavonoids for oral administration already on the market are generally tablets or sachets. Therefore, also the unit doses of the solid pharmaceutical composition of the invention have been designed to contain an equivalent or identical content of two or more microcrystalline flavonoids.

In another embodiment a solid pharmaceutical composition of the invention in the form of tablet typically contains the desired unit dose quantity of two or more microcrystalline flavonoids and variable quantities of the following preferred inactive and auxiliary ingredients :

| Inactive and auxiliary ingredients for the wet granulation | preferred ranges |
|---|---|
| | mg/tablet |
| Microcrystalline cellulose (Avicel 101^{®}) | 55.00 - 70.00 |
| Gelatine (vegetable origin) | 27.00 - 35.00 |
| Sodium starch glycolate | 25.00 - 30.00 |
| Magnesium stearate (vegetable origin) | 3.50 - 4.90 |
| Glycerin | 0.40 - 0.50 |
| Sodium lauryl sulfate | 0.02 - 0.04 |
| 110.92 - 140.44 | |
| Water (final moisture < 2.0 %) (*) | 10.0 - 25.0 |

| | |
|---|---|
| (*) a larger amount of water used for the wet granulation is then evaporated during the granulation-drying step. | |

Therefore, in another embodiment of the invention the total amount of inactive and auxiliary ingredients per tablet may vary from 110.92 mg to 140.44 mg (moisture excluded).

In a further embodiment the tablet of the pharmaceutical composition of the invention is optionally protected by a suitable coating, which is applied according to the conventional methods already well known by the experts in the art. The coating mixture to be applied to the tablet of the invention exhibits the following composition :

| Inactive or auxiliary ingredients for the coating mixture | preferred ranges |
|---|---|
| | mg/tablet |
| Hydroxypropylmethyl cellulose (Type E5) | 3.0 - 10.0 |
| Talc | 2.0 - 10.0 |
| Titanium dioxide | 0.5 - 5.0 |
| Polyethylene glycol (MW : 4000 - 6000) | 0.1 - 1.0 |
| Yellow iron oxide (E 172) | 0.0 - 0.5 |
| Red iron oxide (E 172) | 0.0 - 0.5 |
| 5.6 - 26.5 | |
| White wax (final shining process) | 0.0 - 1.5 |

Therefore, in another embodiment of the invention the total amount per each tablet of coating layer may vary from 5.6 mg to 26.5 mg. The tablet of the invention presents a very reduced size and is very suitable to be easily swallowed. The tablet also exhibits a good stability since it does not undergo degradation processes. Neither changes of colour nor scratches on the surface have been observed even though the tablets are stored for long periods under normal conditions of temperature (25°C ± 2°C) and in conventional containers, such as glass bottles sealed with a suitable stopper or thermosealed blisters commonly available on the market.

Additionally, the coated tablet of the invention exhibits an acceptable organoleptic character, since does not present a bitter taste on the surface, and exhibits also a satisfactory bioavailability as shown in Example 10, below.

In another preferred embodiment the pharmaceutical composition of the present invention is formulated as granule for the preparation of a granulate obtained with the addition of suitable amounts of solid sweetening agents (powder or crystals), such as for instance xylitol, fructose, sorbitol powder, binding agents such as povidone, polyethylene glycol, and flavouring agents alike orange and lemon flavours, mixed in suitable quantities already well known in the art, in order to yield a granulate to be dosed and packed as monodose sealed sachets of the type already available on the market.

The resulting granulate, containing the granule of pharmaceutical composition of the invention, exhibits also a good wettability due to its high specific surface area and therefore yields a prompt drinkable suspension when reconstituted in water before its oral administration. In another embodiment of the invention a pharmaceutical composition is formulated in different compositions suitable for topical administration, which are already well known to skilled artisans. When the pharmaceutical composition of the present invention is formulated as an ointment, the active ingredient may be admixed either to a paraffinic base ointment or to an hydrophilic fatty base. Alternatively, the active ingredient may be formulated as cream in an oil-in-water base at a concentration not less than 4.0 %. The topical formulation of the invention shall exhibit a pH of from 6.5-7.5. The resulting pharmaceutical composition for topical use exhibits high wettability, due to the high specific surface area of the two or more microcrystalline flavonoids used in the invention, so that this preparation for topical use does not present on the skin or on the anal mucosa the uncomfortable "coarse" effect, such as that experienced with some other preparations.

### DESCRIPTION OF PREFERRED EMBODIMENTS

The invention will now be better explained in detail by reference to the following experimental examples, which are described only for presentation of further details rather than for limiting the substance and the scope of the invention itself.

A typical method for the production of microcrystalline (monohydrated) flavonoids with a final specific surface area of more than 9.0 m²/g is given below:
2 Moles of hesperidin and iodine/potassium acetate in industrial methanol (about 5 litres) were reacted (as already described in literature) to yield a reaction mixture of raw flavonoids containing mainly raw diosmin (more than 80 %), hesperidin and other flavonoids, but in quantities above the desired ratio. A more pure solid microcrystalline mixture (raw diosmin more than 90 %, hesperidin, isorhoifolin and others) was filtered after fractioned precipitation in a mixture made alkaline (pH above 8) by means of equimolar quantities of calcium. An aliquot of about 1000 g of the resulting precipitate is suspended again (pH 5-6; t° 15°-35°C) in about 4 L of water and a more pure cake of microcrystalline mixture of flavonoids with high water solvation is precipitated and filtered. In order to yield the desired microcrystalline flavonoids with a final moisture less than 4.0 % (more than 66 % of monohydrated crystals) the cake is vigorously shaken in a vacuum dryer operated at a temperature above 75° C.

928 g of resulting dried microcrystalline mixture, with a water content 2.80 % and a specific surface of 4.85 m²/g, is then jet-milled to yield finally 897 g (1.47 moles) of a product having a specific surface of 10.93 m²/g. The chromatographic analysis according the current European Pharmacopoeia confirmed that the resulting product meets the specifications.

### EXAMPLE 1

A further method for the preparation of microcrystalline flavonoids with a final specific surface area more than 9.0 m²/g.

The manufacturing operations have been carried out in pharmaceutical installations, suitably equipped and operated at the following environmental conditions of relative humidity (R.H.) : 25 % - 40 % and of temperature (t°) : 22°± 3°C.

59.980 Kg of microcrystalline flavonoids (lot 2188), obtained directly from a production process such as that described above and presenting an initial figure of specific surface area of 4.80 m²/g (average value of 3 determinations : 4.93; 4.78; 4.70 m²/g), determined according to BET method, has been passed in nitrogen flow through a conventional fluid-energy mill. After the milling process a mass of 58.884 Kg of two or more microcrystalline flavonoids was obtained. The specific surface area resulted to be 10.67 m²/g (average value of 3 determinations : 9.84; 11.37; 10.81 m²/g), determined according to BET method. Moisture: 2.86 %.

Total yield of the process : 98.17 %.

### EXAMPLE 2

Preparation of 20,000 tablets, each containing 500 mg of two or more microcrystalline flavonoids (specific surface area 10.67 m²/g) obtained from Example 1.

The manufacturing steps were carried out in pharmaceutical installations, suitably equipped for the operations to be performed and operated at the following environmental conditions of relative humidity (R.H.) : 35 % - 50 % and of temperature (t°) : 22°± 3° C. The production operations were protected from direct sun light.

**Each tablet of the composition contains:**

| Active, inert and auxiliary ingredients | mg/tablet |
|---|---|
| Microcrystalline flavonoids (*) | 500.000 |
| Microcrystalline cellulose (Avicel 101^{®}) | 62.000 |
| Gelatine (vegetable origin) | 31.000 |
| Sodium starch glycolate | 27.000 |
| Magnesium stearate (vegetable origin) | 4.426 |
| Glycerin | 0.426 |
| Sodium lauryl sulfate | 0.034 |
| Water (estimated final moisture > 2 %) | 11.490 |
| Theoretical mass of each tablet | 636.370 |

| | |
|---|---|
| (*) expressed as 100.0 % of net active ingredient. | |

Amount of each ingredient necessary for the preparation of a batch of 20,000 tablets having the above theoretical composition per unit dose:

| Active, inert and auxiliary ingredients | weight in Kg |
|---|---|
| Microcrystalline flavonoids (*) | 10.2850 |
| Microcrystalline cellulose (Avicel 101^{®}) | 1.2400 |
| Gelatin (vegetable origin) | 0.6200 |
| Sodium starch glycolate | 0.5400 |
| Magnesium stearate (vegetable origin) | 0.0885 |
| Glycerin | 0.0085 |
| Sodium lauryl sulfate | 0.0007 |
| Water (for wet granulation) (§) | (1.2800) |
| Final moisture : 2.77 % | 0.3650 |
| Total weight | 13.1477 |

| | |
|---|---|
| (*) quantity to meet the 100.0 % of net active ingredient (specific surface area of 10.67 m²/g; moisture 2.85 %); (§) the amount of water used for the wet granulation (1.280 Kg) was then partially eliminated when drying the granulate, to yield a final moisture of the tablet of 2.77 %. | |

The production steps were performed in the following sequence :
step 1) - 1.240 Kg of microcrystalline cellulose (Avicel 101^{®}) and 10.285 Kg of microcrystalline flavonoids fraction were mixed together in a stainless still powder blender.
step 2) -Separately the wetting solution (binding solution) was prepared by mixing together in a suitable container 0.620 Kg of gelatin, 0.0080 Kg of glycerin, 0.0007 Kg of sodium lauryl sulfate and 1.280 Kg of water.
step 3) - The powder mixture from step 1 was then sprayed with the binding solution of step 2 until has obtained the desired consistency.
step 4) - The obtained wet granulate was forced through a 8 mesh screen and then it was dried using a fluid-bed dryer. In order to reduce the static electric charges on the particles, it was desirable to obtain a uniform residual moisture of about 3 %. After drying, the granulate was transformed into the desired particle size by passing it through a small-mesh screen.
step 5) - Finally the dried granulate was added to 0.5400 Kg of sodium starch glycolate and to 0.0885 Kg of magnesium stearate and mixed well to yield an homogeneous powder mixture. By tableting the powder mixture by means of a suitable rotary machine, equipped with oblong punches of the desired shape, size and volume, tablets having an average weight of 657.35 mg have been obtained. The resulting tablets complied with the uniformity of mass test described in the Eur. Ph. 5^{th} Ed. paragraph 2.9.5. The final moisture of the tablet was 2.77 % (w/w) determined according to the semi-micro method described in Eur. Ph. 5^{th} Ed. Method 2.5.12 page 130.

From the above industrial process 18,783 tablets containing 500 mg of microcrystalline flavonoids fraction were obtained.
Final yield of the tableting process: 93.91 %.

### EXAMPLE 3

Preparation of 20,000 tablets, each containing 450 mg of two or more microcrystalline flavonoids (specific surface area 10.67 m²/g).
By using the same quali-quantitative composition of Example 2, as for instance proportional amounts of microcrystalline flavonoids and identical amounts of inert and auxiliary ingredients, by using the same sequence of the manufacturing steps, the same tableting process and installations operated at identical environmental conditions, it was possible to yield 18,435 tablets containing 450 mg of two or more microcrystalline flavonoids used in the invention, having a final residual mixture of 3.09 % (w/w).

Final yield of the tableting process: 92.17 %.

### EXAMPLE 4

Preparation of 15,000 tablets, each containing 600 mg of two or more microcrystalline flavonoids (specific surface area 10.67 m²/g).

By using the same quali-quantitative composition of Example 2, as for instance proportional amounts of microcrystalline flavonoids fraction and identical amounts of inert and auxiliary ingredients, by using the same sequence of the manufacturing steps, the same tableting process and installations operated at identical environmental conditions, it was possible to yield 13,978 tablets containing 600 mg of two or more microcrystalline flavonoids of the invention, having a final residual moisture of 2.94 % (w/w).

Final yield of the tableting process: 93.18 %.

### EXAMPLE 5

Preparation of 18,500 film coated tablets of 450 mg of two or more microcrystalline flavonoids (specific surface area 10.67 m²/g).
The 18,435 tablets resulting from the production process described in Example 3 have been submitted to a coating process in order to improve their palatability when swallowed.

The film coating operations were carried out in pharmaceutical installations suitably equipped with an automatic coating pan and operated at the following environmental conditions of relative humidity (R.H.) : 35 % - 50 % and of temperature (t°): 22° ± 3° C.

Each tablet was coated with a film layer having the following quali-quantitative composition:

| Inert and auxiliary ingredients | mg/coated tablet |
|---|---|
| Hydroxypropylmethyl cellulose (Type E5) | 7.078 |
| Talc | 6.000 |
| Titanium dioxide | 1.362 |
| Polyethylene glycol 6000 | 0.453 |
| Yellow iron oxide (E 172) | 0.166 |
| Red iron oxide (E 172) | 0.055 |
| Theoretical mass of the individual coating | 15.114 |
| White wax (shining process) | 0.800 |
| Theoretical mass of the coating layer | 15.914 |

Amount of each ingredient necessary for the preparation of an excess of coating mixture (for 20,000 tablets) to apply to 18,435 tablets of Example 3:

| Inert and auxiliary ingredients | weight in g |
|---|---|
| Hydroxypropylmethyl cellulose (Type E5) | 141.560 |
| Talc | 120.000 |
| Titanium dioxide | 27.240 |
| Polyethylene glycol 6000 | 9.060 |
| Yellow iron oxide (E 172) | 3.320 |
| Red iron oxide (E 172) | 1.100 |
| Water (eliminated during the process) | 1000.000 |
| Total weight of the coating mixture | 302.280 |
| Total White wax (for the shining process) | 16.000 |

Preparation of the coating mixture for 20,000 tablets : 141.560 g of hydroxypropylmethyl cellulose (type E5), 9.06 g of polyethylene glycol 6000, 120.0 g of talc, 27.240 g of titanium dioxide, 3.320 g of yellow iron oxide (E 172) and 1.1 g of red iron oxide (E 172) were finely suspended in about 1000 ml of water until a homogeneous suspension is obtained. The tablets were placed into a coating pan where the coating mixture is automatically applied to the tablets until the desired coating layer weight was obtained.

A small amount of residual coating mixture was disposed.
Mean weight of the coating layer per tablet: 15.1 mg.
The coated tablets were finally shined by rotating them in a special coating pan with 16.0 g of white wax.

From the coating and shining processes 18,297 coated tablets containing 450 mg of two or more microcrystalline flavonoids were obtained. Final yield of the coating and shining processes: 99.25 %.

### EXAMPLE 6

Preparation of 13,500 film coated tablets of 600 mg of two or more microcrystalline flavonoids (specific surface area 10.67 m²/g).
The film coating operations were carried out in pharmaceutical installations suitably equipped with an automatic coating pan and operated at the following environmental conditions of relative humidity (R.H.) : 35 %-50 % and of temperature (t°) : 22°±3°C.

The 13,978 tablets of the Example 4 were film coated by using proportional amounts of inert and auxiliary ingredients and the same coating and shining processes, as detailed in the previous Example 5. A number of 13,634 coated tablets, each containing 600 mg of two or more microcrystalline flavonoids of the invention, have been obtained. Final yield of the coating and shining processes: 97.53 %.

### EXAMPLE 7

Preparation of 60.19 Kg of granulate containing about 6.94 Kg granules of two or more microcrystalline flavonoids (450 mg per sachet) with specific surface area 10.67 m²/g.

The manufacturing operations were carried out in pharmaceutical installations suitably equipped for the production to be performed and operated at the following environmental conditions of relative humidity (R.H.) : 35 % - 50 % and of temperature (t°) : 22° ± 3°C.

The production operations were protected from direct sun light. Each sachet was designed to contain :

| Active, inert and auxiliary ingredients | mg/sachet |
|---|---|
| Microcrystalline flavonoids (*) | 450.00 |
| Xylitol | 10.00 |
| Orange flavour | 35.00 |
| Theoretical mass of the granules | 495.00 |
| Sorbitol fine powder | 3505.00 |

| | |
|---|---|
| (*) expressed as 100.0 % of net active ingredient. | |

Amount of each ingredient necessary for the preparation of a batch of 60.19 Kg of granulate, corresponding to about 15,000 monodose sachets, each dosed at 450 mg of two or more microcrystalline flavonoids (specific surface area: 10.67 m²/g.)

| Active, inert and auxiliary ingredients | weight in Kg |
|---|---|
| Microcrystalline flavonoids (*) | 6.94 |
| Xylitol | 0.15 |
| Orange flavour | 0.53 |
| Total weight of the granules | 7.62 |
| Sorbitol fine powder | 52.57 |
| Total weight of the granulate | 60.19 |

| | |
|---|---|
| (*) quantity to meet the 100.0 % of net active ingredient (specific surface area of 10.67 m²/g; moisture 2.85 %). | |

The production steps were performed as follows: into a suitable "V" powder mixer were introduced in sequence 52.57 Kg of sorbitol fine powder and then the granules containing a mixture of 6.94 Kg of two or more microcrystalline flavonoids, 0.15 Kg of xylitol and 0.53 Kg of orange flavour. After blending to homogenisation, the resulting granulate was passed to the dose filling operation of the monodose sachets.

The resulting sachets complied with the uniformity of mass test described in the Eur. Ph. 5^{th} Ed. paragraph 2.9.5.

From the above process 14,178 sachets containing each 450 mg of two or more microcrystalline flavonoids were obtained. Final yield of the process: 94.52 %.

### EXAMPLE 8

Preparation of 2,500 tubes of 40 g of fatty cream (100.0 Kg) containing 4.5 % of two or more microcrystalline flavonoids (specific surface area of 10.67 m²/g).

The manufacturing steps were carried out in pharmaceutical installations, suitably equipped for the production of a fatty cream for topical use, operated at the following environmental conditions of relative humidity (R.H.) : 35 % - 50 % and of temperature (t°) : 22° ± 3° C.

100 g of fatty cream for topical use containing:

| Active, inert and auxiliary ingredients | 100g fatty cream |
|---|---|
| Microcrystalline flavonoids (*) | 4.500 |
| Glyceryl monostearate | 12.000 |
| Polyethylene glycol stearate | 2.000 |
| Cetylstearyl alcohol | 2.000 |
| Polysorbate 20 | 1.500 |
| Liquid wax | 1.000 |
| Lanolin | 1.000 |
| Methyl-p-hydroxybenzoate | 0.150 |
| Propyl-p-hydroxybenzoate | 0.050 |
| Perfume essence | 0.500 |
| Water depurated | 75.300 |
| Theoretical mass of fatty cream | 100.000 |

| | |
|---|---|
| (*) expressed as 100.0 % of net active ingredient. | |

Amount of each ingredient necessary for the preparation of a batch of 100.0 Kg of fatty cream, corresponding to about 2,500 tubes of 40 g of fatty cream, having a concentration of 4.5 % of two or more microcrystalline flavonoids of the invention.

| Active, inert and auxiliary ingredients | 100 Kg fatty cream |
|---|---|
| Microcrystalline flavonoids (*) | 4.500 |
| Glyceryl monostearate | 12.000 |
| Polyethylene glycol stearate | 2.000 |
| Cetylstearyl alcohol | 2.000 |
| Polysorbate 20 | 1.500 |
| Liquid wax | 1.000 |
| Lanolin | 1.000 |
| Methyl-p-hydroxybenzoate | 0.150 |
| Propyl-p-hydroxybenzoate | 0.050 |
| Perfume essence | 0.500 |
| Water depurated | 75.300 |
| Total weight of fatty cream | 100.000 |

The preparation of 100.0 Kg of fatty cream was carried out according to the methods well known to skilled artisans experienced with topical preparations. After the filling operations 2,327 tubes of 40 g of fatty cream at 4.5 % of two or more microcrystalline flavonoids fraction were obtained.

Final production yield for the fatty cream : 93.08 %

### EXAMPLE 9

Preparation of 3,000 tubes of 40 g of ointment (120.0 Kg), containing 4.0 % of two or more microcrystalline flavonoids (specific surface area of 10.67 m²/g).

The manufacturing steps were carried out in pharmaceutical installations, suitably equipped for the production of an ointment for topical use, operated at the following environmental conditions of relative humidity (R.H.) : 35 % - 50 % and of temperature (t°) : 22°±3° C.

100 g of ointment for topical use containing:

| Active, inert and auxiliary ingredients | g/100 g ointment |
|---|---|
| Microcrystalline flavonoids (*) | 4.000 |
| Semisynthetic glycerides (Softisan 100) | 12.000 |
| Anionic emulsifying wax (Lanetta SX) | 6.500 |
| Anhydrous lanolin | 1.000 |
| Polysorbate 80 | 1.500 |
| Dimethylpolysiloxane 100 | 4.750 |
| Dimethylpolysiloxane 500 | 4.750 |
| Methyl-p-hydroxybenzoate | 0.140 |
| Propyl-p-hydroxybenzoate | 0.060 |
| Lemon essence | 0.500 |
| Water depurated | 64.800 |
| Theoretical mass of ointment | 100.000 |

| | |
|---|---|
| (*) expressed as 100.0 % of net active ingredient. | |

Amount of each ingredient necessary for the preparation of a batch of 120.0 Kg of ointment, corresponding to about 3,000 tubes of 40 g of preparation having a concentration of 4.0 % of two or more microcrystalline flavonoids:

| Active, inert and auxiliary ingredients | 120.0 Kg ointment |
|---|---|
| Microcrystalline flavonoids (*) | 4.800 |
| Semisynthetic glycerides (Softisan 100) | 14.400 |
| Anionic emulsifying wax (Lanetta SX) | 7.800 |
| Anhydrous lanolin | 1.200 |
| Polysorbate 80 | 1.800 |
| Dimethylpolysiloxane 100 | 5.700 |
| Dimethylpolysiloxane 500 | 5.700 |
| Methyl-p-hydroxybenzoate | 0.168 |
| Propyl-p-hydroxybenzoate | 0.072 |
| Lemon essence | 0.600 |
| Water depurated | 77.760 |
| Total weight of ointment | 120.000 |

The preparation of 120.0 Kg of ointment was carried out according to the methods well known to skilled artisans experienced with topical preparations. After the filling operations 2,812 tubes of 40 g of ointment at 4.0 % of the two or more microcrystalline flavonoids were obtained. Final production yield for the ointment: 93.93%

### EXAMPLE 10

Pilot pharmacokinetic study following to one single oral dose administration to volunteers of a film coated tablet of Example 6 containing 600 mg of two or more microcrystalline flavonoids (specific surface area of 10.67 m²/g).

Five healthy volunteers (2 males and 3 females), with an age ranging from 20 to 45 years and a body weight comprised into an interval from 52 to 68 Kg, were admitted to the study after a complete medical screening.

Each fasting subject received orally in one single dose 1 film coated tablet of 600 mg of two or more microcrystalline flavonoids, corresponding to about 10 mg/Kg body weight.

Blood samples were collected from the subjects by using known techniques at intervals of 0.5,1, 2, 4, 8, 12, 24 and 48 hours after oral administration and centrifuged immediately at 3,000 g. Each plasma sample was individually withdrawn, separated from the corpuscular fraction and stored at -20 °C until its analysis.

The determination of the content of the two or more microcrystalline flavonoids, was carried out by using the following method:
- addition of 1 ml of DMSO to 1 ml of plasma sample and shaking for 10 minutes;
- centrifugation at 2.5 rpm for 5 minutes;
- separation of the organic phase and evaporation of the same to dryness by nitrogen;
- addition of 50 µl of methanol to the dry residue;
- injection of 20 µl of the above solution into the HPLC apparatus, equipped with a PKB 100 column (25 cm x 2.1 mm), using as mobile phase a mixture of methanol/acetic acid/water (40:15:45) and a flow rate of 1.0 ml/min;
- the elute was monitored for absorbance at 345 nm;
- the retention time of the microcrystalline flavonoids was about 1.3 minutes.

The following parameters have been investigated for the elaboration of the pharmacokinetic parameters :
- Cₘₐₓ :: maximum plasma concentration of the two or more microcrystalline flavonoids;
- Tₘₐₓ: time to reach the maximum plasma concentration;
- AUC :: area under the plasma concentration-time curve within the dosing interval, calculated by the trapezoidal rule;
- MRT :: mean residence time;
- TCI :: total body clearance resulting by ratio of oral dose (D) and AUC;
- V_{d} :: volume of distribution.

The results are summarized in the following Table 1.

**Table 1**

| PARAMETERS | SUBJECT N. | | | | | MEAN VALUE | SD |
|---|---|---|---|---|---|---|---|
| | 1 | 2 | 3 | 4 | 5 | | |
| C_{MAX} (ng/ml) | 503 | 510 | 315 | 326 | 435 | 417,80 | 93,61 |
| T _{MAX} (h) | 35,8 | 44,8 | 23 | 27,5 | 28 | 31,82 | 8,59 |
| MRT (h) | 46 | 48,5 | 26,8 | 29,8 | 32,2 | 36,66 | 9,89 |
| AUC (0-48h) ng/ml h | 7200 | 7400 | 4300 | 4600 | 4700,8 | 5640,16 | 1524,0 |
| TCl (Lt/h) | 1,77 | 1,85 | 0,8 | 1 | 1,2 | 1,32 | 0,47 |
| Vd (Lt) | 68 | 72,3 | 53 | 56,8 | 60 | 60,02 | 7,97 |

## Claims

1. A pharmaceutical composition comprising an active ingredient comprising two or more flavonoids in microcrystalline form, wherein the two or more microcrystalline flavonoids comprise diosmin and at least one other flavonoid; and further wherein the two or more microcrystalline flavonoids exhibit the following characterizing parameters :
a specific surface area of 4 to 18 m²/g, according to BET determination;
an F/D (F=other flavonoids/D=diosmin) (weight/weight) ratio of from 0.03 to 0.1; and
a moisture content of less than 4.0 % by weight.

2. A pharmaceutical composition according to claim 1, wherein the two or more microcrystalline flavonoids exhibit a specific surface area of from 9 to 12 m²/g, according to BET determination.

3. A pharmaceutical composition according to claim 1 or 2, in the form of a tablet or granule for oral administration, wherein the amount of the two or more microcrystalline flavonoids represents not less than 70.0 % (w/w) of the weight of the tablet or granule.

4. A pharmaceutical composition according to claim 1 or 2, in the form of fatty cream or ointment for external topical use, wherein the amount of the two or more microcrystalline flavonoids represents not less than 4.0 % (w/w) of the weight of the fatty cream or ointment.

5. A pharmaceutical composition according to any preceding claim wherein the two or more microcrystalline flavonoids exhibit X-ray powder diffractogram (XRD) patterns with peaks of relative intensity strong (s) at about 15.32, 19.68, 21.70, medium (m) at about 11.90, 15.87, 21.33, 24.68 and diffuse (d) at about 26.35, 27.72 and 28.65.

6. A pharmaceutical composition according to any one of claims 1 to 5 wherein the at least one other microcrystalline flavonoid is selected from one or more of isorhoifolin, linarin, diosmetin, hesperidin, quercitin and rutin.

7. A process for the preparation of a pharmaceutical composition according to any one of claims 1 to 6 in the form of granules, comprising passing the two or more microcrystalline flavonoids, together with selected inert and auxiliary ingredients, through a wet granulation procedure and then further steps to homogeneously admix, spray or knead into a suitable compression mixture.

8. A process according to claim 7 wherein the compression mixture to be punched into tablets, in addition to the at least two microcrystalline flavonoids, contains other inert and auxiliary ingredients in a specific weight range (expressed as mg/tablet):
| | |
|---|---|
| Microcrystalline cellulose | 55.0 - 70.0 mg |
| Gelatine | 27.0 - 35.0 mg |
| Sodium starch glycolate | 25.0 - 30.0 mg |
| Magnesium stearate | 3.50 - 4.90 mg |
| Glycerin | 0.40 - 0.50 mg |
| Sodium lauryl sulfate | 0.02 - 0.04 mg |
being the total weight of the inert and auxiliary ingredients for each tablet ranging from 110.92 mg to 140.44 mg.

9. A process for the preparation of the compression mixture of claim 8 **characterized in that** the content of the disintegrating agent sodium starch glycolate is more than 15.0 % of the total weight of the inactive and auxiliary ingredients.

10. A process according to any one of claims 7 to 9 comprising the following production sequence:
(a) incorporate homogeneously the microcrystalline cellulose into the two or more microcrystalline flavonoids (powder mixture);
(b) prepare separately the binding solution by dissolving gelatine, glycerin and sodium lauryl sulfate in a convenient quantity of water;
(c) conveniently spray or wet or knead the powder mixture (a) with the binding solution (b) to yield the wet granulate to be conveniently sieved into granules of adequate consistency and size;
(d) dry the wet granulate on a fluid-bed desiccator (moisture more than 2.0 %) to be conveniently milled and sieved; and
(e) homogeneously blend the dry granules (d) with sodium starch glycolate to yield the compression mixture to be punched into tablets.

11. A process as claimed in any one of claims 7 to 10 wherein the resulting tablet exhibits a residual moisture content of more than 2.0 % (w/w).

12. A process to apply an optional coating on the surface of the tablet obtained according to any one of claims 7 to 11 comprising using an aqueous coating suspension that comprises the following ingredients in a specific weight range (expressed as mg/tablet):
| | |
|---|---|
| Hydroxypropylmethyl cellulose | 3.0 -10.0 mg |
| Talc | 2.0 - 10.0 mg |
| Titanium dioxide | 0.5 - 5.0 mg |
| Polyethylene glycol | 0.1 - 1.0 mg |
| Yellow iron oxide (E 172) | 0.0 - 0.5 mg |
| Red iron oxide (E 172) | 0.0 - 0.5 mg |
| White wax (final shining process) | 0.0 - 1.5 mg |
being the total weight of the coating for each tablet ranging from 5.6 mg to 26.5 mg.

13. A process for the preparation of a pharmaceutical composition in the form of granulate to fill monodose sachets, **characterized in that** it is prepared by admixing the granules formed according to claim 7 with suitable amounts of solid sweetening agents and flavouring agents.

14. A process for the preparation of a pharmaceutical composition according to any one of claims 1 and 4 in the form of fatty cream or ointment comprising admixing 4.0 % (w/w) of the two or more microcrystalline flavonoids with convenient quantities of suitable inert and auxiliary ingredients to yield a physiological range of pH comprised between 6.5 and 7.5.

15. A medicament comprising two or more microcrystalline flavonoids according to the any of the preceding claims **characterized in that** the medicament may be formulated as tablet, film-coated tablet, granule and granulate for oral administration, or formulated as fatty cream or ointment for external topical use, said medicament exhibiting a remarkable patient compliance and an improved wettability, absorption rate and bioavailability.

16. A medicament comprising two or more microcrystalline flavonoids in accordance to any of the preceding claims wherein the medicament exhibits venotonic and vasculoprotective effects, particularly useful for the treatment of chronic venous disorders.

17. A method for the preparation of a microcrystalline flavonoid for use a pharmaceutical composition as claimed in claim 1, comprising the steps:
a. reacting hesperidin with iodine/potassium acetate in methanol;
b. increasing the amount of diosmin in the reaction product obtained from step a) by fractioned precipitation; and drying the product obtained from step b).

## Patentansprüche

1. Pharmazeutische Zusammensetzung, die einen aktiven Bestandteil enthält, welcher zwei oder mehr Flavonoide in mikrokristalliner Form enthält, wobei die zwei oder mehr mikrokristallinen Flavonoide Diosmin und mindestens ein weiteres Flavonoid umfassen; und wobei die zwei oder mehr mikrokristallinen Flavonoide ferner folgende kennzeichnende Eigenschaften aufweisen:
eine spezifische Oberfläche von 4 bis 18 m²/g nach BET-Messung;
ein (Gewicht/Gewicht-)Verhältnis F/D (F = andere Flavonoide / D = Diosmin) von 0,03 zu 0,1; und
einen Feuchtegehalt von weniger als 4,0 Gewichtsprozent.

2. Pharmazeutische Zusammensetzung nach Anspruch 1, wobei die zwei oder mehr mikrokristallinen Flavonoide eine spezifische Oberfläche von 9 bis 12 m²/g nach BET-Messung aufweisen.

3. Pharmazeutische Zusammensetzung nach Anspruch 1 oder 2 in Form einer Tablette oder einer Granalie zu oralen Verabreichung, wobei die Menge der zwei oder mehr mikrokristallinen Flavonoide nicht weniger als 70,0 % (w/w) des Gewichts der Tablette bzw. der Granalie ausmacht.

4. Pharmazeutische Zusammensetzung nach Anspruch 1 oder 2 in Form einer Fettcreme oder -salbe zur äußerlichen lokalen Anwendung, wobei die Menge der zwei oder mehr mikrokristallinen Flavonoide nicht weniger als 4,0 % (w/w) des Gewichts der Fettcreme bzw. -salbe ausmacht.

5. Pharmazeutische Zusammensetzung nach einem der vorstehenden Ansprüche, wobei die zwei oder mehr mikrokristallinen Flavonoide ein Röntgen-Pulverdiffraktogramm (XRD) -Muster mit Spitzen einer relativen Intensität stark (s) bei etwa 15,32, 19,68, 21,70, mittel (m) bei etwa 11,90, 15,87, 21,33, 24,68 und diffus (d) bei etwa 26,35, 27,72 und 28,65 aufweisen.

6. Pharmazeutische Zusammensetzung nach einem der Ansprüche 1 bis 5, wobei das mindestens eine andere mikrokristalline Flavonoid aus einem oder mehreren von Isorhoifolin, Linarin, Diosmetin, Hesperidin, Quercitin und Rutin ausgewählt ist.

7. Verfahren zur Herstellung einer pharmazeutischen Zusammensetzung nach einem der Ansprüche 1 bis 6 in Form von Granalien, das umfasst: die zwei oder mehr mikrokristallinen Flavonoide zusammen mit ausgewählten inerten und Hilfsbestandteilen einem Nassgranulierungsverfahren zu unterziehen, und anschließend weitere Schritte, um sie homogen zu einer geeigneten verdichteten Mischung zu mischen, sprühen oder kneten.

8. Verfahren nach Anspruch 7, wobei die verdichtete Mischung, die zu Tabletten gestanzt werden soll, zusätzlich zu den mindestens zwei mikrokristallinen Flavonoiden weitere inerte und Hilfsbestandteile in einem spezifischen Gewichtsbereich (angegeben in mg/Tablette) beinhaltet:
| | |
|---|---|
| Mikrokristalline Cellulose | 55,0 - 70,0 mg |
| Gelatine | 27,0 - 35,0 mg |
| Natriumstärkeglycolat | 25,0 - 30,0 mg |
| Magnesiumstearat | 3,50 - 4,90 mg |
| Glycerin | 0,40 - 0,50 mg |
| Natrium-Laurylsulfat | 0,02 - 0,04 mg |
wobei das Gesamtgewicht der inerten und Hilfsbestandteile bei jeder Tablette im Bereich von 110,92 mg bis 140,44 mg liegt.

9. Verfahren zur Herstellung der verdichteten Mischung nach Anspruch 8, **dadurch gekennzeichnet, dass** der Gehalt an dem Zerfallbeschleuniger Natriumstärkeglycolat mehr als 15,0 % des Gesamtgewichts der inerten und Hilfsbestandteile beträgt.

10. Verfahren nach einem der Ansprüche 7 bis 9, das den folgenden Produktionsablauf umfasst:
(a) die mikrokristalline Cellulose homogen in die zwei oder mehr mikrokristallinen Flavonoide einzubauen (Pulvermischung);
(b) die Bindelösung separat herzustellen durch Auflösen von Gelatine, Glycerin und Natrium-Laurylsulfat in einer geeigneten Menge Wasser;
(c) die Pulvermischung (a) in geeigneter Weise mit der Bindelösung (b) zu besprühen oder zu benetzen oder zu verkneten, um das Nassgranulat zu erhalten, das in dienlicher Weise zu Granalien geeigneter Konsistenz und Größe gesiebt wird;
(d) das Nassgranulat auf einem Wirbelschichttrockner zu trocknen (Feuchtegehalt über 2,0 %), damit es in geeigneter Weise gemahlen und gesiebt werden kann; und
(e) die trockenen Granalien (d) homogen mit Natriumstärkeglycolat zu mischen, um die in Tabletten zu stanzende verdichtete Mischung zu erhalten.

11. Verfahren nach einem der Ansprüche 7 bis 10, wobei die erhaltene Tablette einen Restfeuchtegehalt von über 2,0 % (w/w) aufweist.

12. Verfahren zum Aufbringen einer Beschichtung nach Wahl auf die Oberfläche der Tablette, die nach einem der Ansprüche 7 bis 11 erhalten wird, das die Verwendung einer wässrigen Beschichtungssuspension umfasst, die die folgenden Bestandteile in einem spezifischen Gewichtsbereich (angegeben in mg/Tablette) beinhaltet:
| | |
|---|---|
| Hydroxypropylmethylcellulose | 3,0 -10,0 mg |
| Talkum | 2,0 - 10,0 mg |
| Titandioxid | 0,5 - 5,0 mg |
| Polyethylenglycol | 0,1 -1,0 mg |
| Eisenoxidgelb (E 172) | 0,0 - 0,5 mg |
| Eisenoxidrot (E 172) | 0,0 - 0,5 mg |
| Weißes Wachs (abschließende Politur) | 0,0 - 1,5 mg |
wobei das Gesamtgewicht der Beschichtung pro Tablette im Bereich von 5,6 mg bis 26,5 mg liegt.

13. Verfahren zur Herstellung einer pharmazeutischen Zusammensetzung in Form eines Granulats zur Befüllung von Einzeldosis-Beuteln, **dadurch gekennzeichnet, dass** sie hergestellt wird, indem die nach Anspruch 7 hergestellten Granalien mit geeigneten Mengen fester Süßungsmittel und Geschmacksstoffe vermischt werden.

14. Verfahren zur Herstellung einer pharmazeutischen Zusammensetzung nach einem der Ansprüche 1 bis 4 in Form einer Fettcreme oder -salbe, das umfasst, 4,0 % (w/w) der zwei oder mehr mikrokristallinen Flavonoide mit dienlichen Mengen geeigneter inerter und Hilfsbestandteile zu mischen, um einen physiologischen pH-Wert im Bereich von 6,5 bis 7,5 zu erhalten.

15. Arzneimittel beinhaltend zwei oder mehr mikrokristalline Flavonoide nach einem der vorstehenden Ansprüche, **dadurch gekennzeichnet, dass** das Arzneimittel als Tablette, Filmtablette, Granalie bzw. Granulat zur oralen Verabreichung oder als Fettcreme oder
- salbe zur äußerlichen lokalen Anwendung formuliert sein kann, wobei das Arzneimittel eine bemerkenswerte Patientencompliance und eine verbesserte Benetzbarkeit, Absorptionsrate und Bioverfügbarkeit aufweist.

16. Arzneimittel beinhaltend zwei oder mehr mikrokristalline Flavonoide nach einem der vorstehenden Ansprüche, wobei das Arzneimittel venentonisierende und vaskuloprotektive Wirkungen aufweist, die insbesondere zur Behandlung von chronischen Venenerkrankungen geeignet sind.

17. Verfahren zur Herstellung eines mikrokristallinen Flavonoids zur Verwendung in einer pharmazeutischen Zusammensetzung wie in Anspruch 1 angegeben, das die Schritte umfasst:
a. Hesperidin mit Iod/Kaliumacetat in Methanol zur Reaktion zu bringen;
b. die Menge an Diosmin in dem Reaktionsprodukt, das in Schritt a) erhalten wurde, durch fraktionierte Präzipitation zu erhöhen; und das in Schritt b) erhaltene Produkt zu trocknen.

## Revendications

1. Une composition pharmaceutique comprenant un principe actif comprenant deux ou plus flavonoïdes sous forme microcristalline, où les deux ou plus flavonoïdes microcristallins comprennent de la diosmine et au moins un autre flavonoïde, et où en outre les deux ou plus flavonoïdes microcristallins présentent les paramètres caractéristiques suivants :
une surface spécifique de 4 à 18 m²/g, selon la détermination BET,
un rapport F/D (F = autres flavonoïdes/D = diosmine) (poids/poids) de 0,03 à 0,1, et
une teneur en eau de moins de 4,0% en poids.

2. Une composition pharmaceutique selon la revendication 1, où les deux ou plus flavonoïdes microcristallins présentent une surface spécifique de 9 à 12 m²/g, selon la détermination BET.

3. Une composition pharmaceutique selon la revendication 1 ou 2, sous la forme d'un comprimé ou d'une granule pour administration orale, où le volume des deux ou plus flavonoïdes microcristallins représente pas moins de 70,0% (p/p) du poids du comprimé ou de la granule.

4. Une composition pharmaceutique selon la revendication 1 ou 2, sous la forme d'une crème grasse ou d'un onguent pour usage topique externe, où le volume des deux ou plus flavonoïdes microcristallins représente pas moins de 4,0% (p/p) du poids de la crème grasse ou de l'onguent.

5. Une composition pharmaceutique selon l'une quelconque des revendications précédentes où les deux ou plus flavonoïdes microcristallins présentent des phénotypes de diffractogramme de rayons X sur poudre (XRD) avec des crêtes d'intensité relative forte (s) à environ 15,32, 19,68, 21,70, moyenne (m) à environ 11,90, 15,87, 21,33, 24,68 et diffuse (d) à environ 26,35, 27,72 et 28,65.

6. Une composition pharmaceutique selon l'une quelconque des revendications 1 à 5 où le au moins un autre flavonoïde microcristallin est sélectionné parmi un ou plusieurs des éléments suivants : isorhoifoline, linarine, diosmétine, hespéridine, quercitine et rutine.

7. Un processus destiné à la préparation d'une composition pharmaceutique selon l'une quelconque des revendications 1 à 6 sous la forme de granules, comprenant le passage des deux ou plus flavonoïdes microcristallins, conjointement avec des ingrédients inertes et auxiliaires sélectionnés, par une procédure de granulation par voie humide et ensuite par des opérations complémentaires destinées à les incorporer, pulvériser ou malaxer de manière homogène en un mélange à compression adapté.

8. Un processus selon la revendication 7 où le mélange à compression destiné à être poinçonné en comprimés, en plus des au moins deux flavonoïdes microcristallins, contient d'autres ingrédients inertes et auxiliaires dans une plage de poids spécifique (exprimée en mg/comprimé) :
| | |
|---|---|
| Cellulose microcristalline | 55,0 - 70,0 mg |
| Gélatine | 27,0 - 35,0 mg |
| Glycolate d'amidon de sodium | 25,0 - 30,0 mg |
| Stéarate de magnésium | 3,50 - 4,90 mg |
| Glycérine | 0,40 - 0,50 mg |
| Laurylsulfate de sodium | 0,02 - 0,04 mg |
étant le poids total des ingrédients inertes et auxiliaires pour chaque comprimé allant de 110,92 mg à 140,44 mg.

9. Un processus destiné à la préparation du mélange à compression selon la revendication 8 **caractérisé en ce que** la teneur de l'agent désintégrant glycolate d'amidon de sodium est supérieure à 15,0% du poids total des ingrédients inertes et auxiliaires.

10. Un processus selon l'une quelconque des revendications 7 à 9 comprenant la séquence de production suivante :
(a) incorporer de manière homogène la cellulose microcristalline dans les deux ou plus flavonoïdes microcristallins (mélange pulvérulent),
(b)préparer séparément la solution de liaison en dissolvant de la gélatine, de la glycérine et du laurylsulfate de sodium dans un volume d'eau approprié,
(c) pulvériser, mouiller ou pétrir comme il convient le mélange pulvérulent (a) avec la solution de liaison (b) de façon à amener le granulat humide à être tamisé comme il convient en granules de taille et de consistance appropriées,
(d)sécher le granulat humide sur un dessiccateur à lit fluidifié (teneur en eau supérieure à 2,0%) de façon à être malaxé et tamisé comme il convient, et
(e) mélanger de manière homogène les granules sèches (d) avec du glycolate d'amidon de sodium de façon à amener le mélange à compression à être poinçonné en comprimés.

11. Un processus selon l'une quelconque des revendications 7 à 10 où le comprimé résultant présente une teneur en eau résiduelle supérieure à 2,0% (p/p).

12. Un processus destiné à appliquer un enrobage optionnel sur la surface du comprimé obtenu selon l'une quelconque des revendications 7 à 11 comprenant l'utilisation d'une suspension d'enrobage aqueuse qui comprend les ingrédients suivants dans une plage de poids spécifique (exprimée en mg/comprimé) :
| | |
|---|---|
| Hydroxypropylméthylcellulose | 3,0 - 10,0 mg |
| Talc | 2,0 - 10,0 mg |
| Dioxyde de titane | 0,5 - 5,0 mg |
| Glycol polyéthylénique | 0,1 - 1,0 mg |
| Oxyde de fer jaune (E 172) | 0,0 - 0,5 mg |
| Oxyde de fer rouge (E 172) | 0,0 - 0,5 mg |
| Cire blanche (processus de lustrage final) | 0,0 - 1,5 mg |
étant le poids total de l'enrobage pour chaque comprimé allant de 5,6 mg à 26,5 mg.

13. Un processus destiné à la préparation d'une composition pharmaceutique sous la forme de granulés destinée à remplir des sachets monodoses, **caractérisé en ce qu'**elle est préparée en incorporant les granules formées selon la revendication 7 à des quantités adaptées d'agents édulcorants solides et d'agents aromatisants.

14. Un processus destiné à la préparation d'une composition pharmaceutique selon l'une quelconque des revendications 1 et 4 sous la forme d'une crème grasse ou d'un onguent comprenant l'incorporation de 4,0% (p/p) des deux ou plus flavonoïdes microcristallins à des quantités qui conviennent d'ingrédients inertes et auxiliaires adaptés de façon à produire une plage physiologique de pH comprise entre 6,5 et 7,5.

15. Un médicament comprenant deux ou plus flavonoïdes microcristallins selon l'une quelconque des revendications précédentes **caractérisé en ce que** le médicament peut être formulé sous la forme d'un comprimé, d'un comprimé pelliculé, d'une granule et d'un granulé pour administration orale, ou formulé sous la forme d'une crème grasse ou d'un onguent pour usage topique externe, ledit médicament présentant une observance du patient remarquable et une mouillabilité, une vitesse d'absorption et une biodisponibilité améliorées.

16. Un médicament comprenant deux ou plus flavonoïdes microcristallins selon l'une quelconque des revendications précédentes où le médicament présente des effets veinotoniques et vasculoprotecteurs particulièrement utiles pour le traitement de troubles veineux chroniques.

17. Un procédé destiné à la préparation de flavonoïdes microcristallins destinés à une utilisation dans une composition pharmaceutique selon la revendication 1 comprenant les opérations suivantes :
a. réaction d'hespéridine avec de l'acétate de potassium/iode dans du méthanol,
b. augmentation de la quantité de diosmine dans le produit de réaction obtenu à l'opération a) par précipitation fractionnée, et séchage du produit obtenu à l'opération b).
